# EUROPEAN PATENT APPLICATION

(11) **EP 3 936 152 A1**
(43) Date of publication of application: **12.01.2022**
(21) Application number: 20766490.5
(22) Date of filing: 04.03.2020
(51) Int. Cl.: A61K 48/00, A61P 25/00, A61P 27/02, A61P 27/06, A61P 43/00, C07K 14/705, C12N 5/0793, C12N 15/12, C12N 15/62, C12N 15/864, A61K 38/16, A61K 31/7088, A61K 35/30, A61K 35/76

(54) **NUCLEIC ACID CONSTRUCT ENCODING TRK FRAGMENT AND USE THEREOF**

(30) Priority: 04.03.2019 JP 2019038503
(71) Applicant: Tokyo Metropolitan Institute of Medical Science, Tokyo 156-8506 (JP)
(72) Inventor: NAMEKATA, Kazuhiko, Tokyo 156-8506 (JP); HARADA, Takayuki, Tokyo 156-8506 (JP)
(74) Representative: Graf von Stosch Patentanwaltsgesellschaft mbH
(86) International application number: PCT/JP2020/009259
(87) International publication number: WO 2020/179844

(57) **Abstract**

The present invention provides: a novel nucleic acid construct encoding a functional fragment of a Trk family member; and use of the nucleic acid construct. A nucleic acid construct in accordance with an embodiment of the present invention encodes a fusion polypeptide including an intracellular domain of Trk and a membrane localization sequence.

## Description

### Technical Field

The present invention relates to a nucleic acid construct encoding a functional fragment of a Trk family member and to use of the nucleic acid construct.

### Background Art

Nerve growth factor (NGF), brain-derived neurotrophic factor (BDNF), neurotrophin-3 (NT-3), NT-4/5, and the like are known as neurotrophic factors which play important roles in neural cell survival, axonal outgrowth, generation of a central nervous system, and the like (Non-patent Literature 1).

TrkB is known as a high-affinity receptor for the above BDNF. Typically, an extracellular domain of TrkB forms a dimer so that TrkB can receive BDNF. However, there is also a truncated form of TrkB that lacks a tyrosine kinase domain in a cell, and the function of the truncated form of TrkB is not fully understood (Non-patent Literature 2).

The inventors of the present invention have studied neurotrophic factors for years and have reported that control of signaling between glia cells and signaling between a glia cell and a neuron is important for neuroprotection (Non-patent Literatures 3 to 5).

Further, as the inventors continued the study of neuroprotection and axon regeneration by TrkB signaling, the inventors have been focusing on the functions and the like of Dock3, which is a guanine nucleotide exchange factor (GEF) activated by TrkB signaling (Non-patent Literatures 6 to 9).

### Citation List

### [Patent Literature]

[Patent Literature 1] International Publication WO2018/185468 (International Publication Date: October 11, 2018)

### [Non-patent Literature]

[Non-patent Literature 1] Park H, Poo MM. Neurotrophin regulation of neural circuit development and function. Nat Rev Neurosci 14(1): 7-23, 2013.
[Non-patent Literature 2] Fenner BM. Truncated TrkB: beyond a dominant negative receptor. Cytokine Growth Factor Rev 23(1-2): 15-24, 2012.
[Non-patent Literature 3] Harada T, Harada C, Nakayama N, Okuyama S, Yoshida K, Kohsaka S, Matsuda H, Wada K. Modification of glial-neuronal cell interactions prevents photoreceptor apoptosis during light-induced retinal degeneration. Neuron 26(2): 533-541, 2000.
[Non-patent Literature 4] Harada T, Harada C, Kohsaka S, Wada E, Yoshida K, Ohno S, Mamada H, Tanaka K, Parada LF, Wada K. Microglia-Muller glia cell interactions control neurotrophic factor production during light-induced retinal degeneration. J Neurosci 22(21): 9228-9236, 2002.
[Non-patent Literature 5] Harada C, Guo X, Namekata K, Kimura A, Nakamura K, Tanaka K, Parada LF, Harada T. Glia- and neuronspecific functions of TrkB signalling during retinal degeneration and regeneration. Nat Commun 2: 189, 2011.
[Non-patent Literature 6] Namekata K, Harada C, Taya C, Guo X, Kimura H, Parada LF, Harada T. Dock3 induces axonal outgrowth by stimulating membrane recruitment of the WAVE complex. Proc Natl Acad Sci U S A 107(16): 7586-7591, 2010.
[Non-patent Literature 7] Namekata K, Harada C, Guo X, Kimura A, Kittaka D, Watanabe H, Harada T. Dock3 stimulates axonal outgrowth via GSK-3β-mediated microtubule assembly. J Neurosci 32(1): 264-274, 2012.
[Non-patent Literature 8] Namekata K, Kimura A, Kawamura K, Harada C, Harada T. Dock GEFs and their therapeutic potential: neuroprotection and axon regeneration. Prog Retin Eye Res 43: 1-16, 2014.
[Non-patent Literature 9] Kazuhiko NAMEKATA, Takayuki HARADA. Shinkei Jikusaku no Saisei ni okeru Dock3 no Kino (Function of Dock3 in Regeneration of Neural Axons). SEIKAGAKU (Biochemistry) 84(5), 368-373, 2012.

### Summary of Invention

### Technical Problem

As described above, the important roles of TrkB in neuroprotection and the like are beginning to be understood from various viewpoints.

However, it is difficult to induce high-level expression of TrkB with use of a genetic vector having a full-length TrkB gene incorporated therein. This is because the full-length TrkB gene has a relatively large size, so that a workable size of a promoter and the like are restricted.

Further, in treatment of a nerve-related disease, it is considered necessary to not only cause overexpression of TrkB but also periodically administer BDNF at the same time. As such, no study of gene therapy utilizing TrkB has been put into practice.

A recent report shows an expression vector that has incorporated therein a gene encoding a fusion protein of BDNF and TrkB (Patent Literature 1). However, considering the molecular size of the fusion protein, it is presumed that the expression level is not very high.

The present invention has been accomplished in light of the above problems. An objective of the present invention is to provide a novel nucleic acid construct encoding a functional fragment of a Trk family member, and use of the nucleic acid construct.

### Solution to Problem

In order to attain the objective, the present invention encompasses the following aspects.
(i) A nucleic acid construct encoding a fusion polypeptide, the fusion polypeptide including: an intracellular domain of Trk; and a membrane localization sequence.
(ii) A method for cell activation, including the step of causing an intracellular domain of Trk to be localized at a cell membrane via a membrane localization sequence in a cell.
(iii) A fusion polypeptide including: an intracellular domain of Trk; and a membrane localization sequence.

### Advantageous Effects of Invention

According to an aspect of the present invention, it is possible to provide a novel nucleic acid construct encoding a functional fragment of a Trk family member, use of the nucleic acid construct, and the like.

### Brief Description of Drawings

Fig. 1 is a view illustrating a structure of constitutive active TrkB (CA-TrkB).
Fig. 2 is a view illustrating an adeno-associated virus vector prepared in Example 1.
Fig. 3 is a view illustrating results (an expression level of CA-TrkB) of Example 1.
Fig. 4 is a view illustrating other results (intracellular signaling by CA-TrkB) of Example 1.
Fig. 5 is a view illustrating results (protection of retinal ganglion cells by CA-TrkB) of Example 2.
Fig. 6 is a view illustrating results (optic nerve axon regeneration by CA-TrkB) of Example 2.
Fig. 7 is a view illustrating results (protection of retinal ganglion cells by CA-TrkB with use of glaucoma model mice) of Example 3.
Fig. 8 is a view illustrating results (examination of signaling with use of mutants of CA-TrkB) of Example 4.
Fig. 9 is a view relating to Example 5 and illustrating structures of constitutive active TrkA and TrkC and results (intracellular signaling by CA-TrkA and CA-TrkC) of Example 5.
Fig. 10 is a view illustrating a base sequence of a gene fragment encoding iSH-TrkB in an Example.
Fig. 11 is a view illustrating a base sequence of a gene fragment encoding CA-TrkA in an Example.
Fig. 12 is a view illustrating a base sequence of a gene fragment encoding CA-TrkC in an Example.
Fig. 13 is a view illustrating results (measurement of a visual evoked potential) of Example 6.

### Description of Embodiments

### [Definitions of terms and the like]

As used herein, the term "polynucleotide" can be interpreted as meaning "nucleic acid" or "nucleic acid molecule". The term "polynucleotide", unless otherwise specified, includes in its scope a polynucleotide that contains a known analog of a naturally-occurring nucleotide capable of functioning similarly to the naturally-occurring nucleotide. The term "base sequence" can be interpreted as meaning "nucleic acid sequence" or "nucleotide sequence". Unless otherwise indicated, the term "base sequence" is intended to mean a deoxyribonucleotide sequence or a ribonucleotide sequence. The polynucleotide may have a single-stranded structure or a double-stranded structure, and may be a sense strand or an antisense strand in the case of a single strand.

As used herein, the term "gene" refers to a "polynucleotide" encoding a protein.

As used herein, the term "expression regulatory region" of a gene refers to a "polynucleotide" that regulates expression of the gene. Examples of the "expression regulatory region" include a promoter region and an enhancer region.

As used herein, the term "expression cassette" refers to an expression unit including (i) an expression regulatory region that is functional in an expression host and (ii) a polynucleotide that is operably linked to the expression regulatory region. In the expression cassette, the polynucleotide is preferably a gene or a gene fragment. An example of the expression cassette is an expression unit in which the above expression regulatory region is linked to the above polynucleotide in a genetically engineered manner. The term "operably linked" refers to a state in which expression of a polynucleotide is regulated with use of an expression regulatory sequence. The expression cassette may be in the form of an expression vector.

As used herein, the term "polypeptide" is interchangeable with "protein". A "polypeptide" includes a structure of amino acids linked by a peptide bond. A "polypeptide" may further include a structure of a sugar chain or an isoprenoid group and the like. The term "polypeptide", unless otherwise specified, includes in its scope a polypeptide that contains a known analog of a naturally-occurring amino acid having a function as well as the naturally-occurring amino acid.

### [1. Nucleic acid construct]

A nucleic acid construct in accordance with an embodiment of the present invention is a nucleic acid construct encoding a fusion polypeptide including an intracellular domain of Trk and a membrane localization sequence. The present invention has been made based on the surprising knowledge that causing the nucleic acid construct to be expressed in a cell enables activation of an intracellular signaling pathway downstream of Trk without administration of a neurotrophic factor (neurotrophin). That is, the present invention relates to a novel cell activation technique of causing the intracellular domain of Trk to be localized at a cell membrane via the membrane localization sequence of the fusion polypeptide encoded by the nucleic acid construct.

The nucleic acid construct in accordance with the present embodiment, for example, provides effects typified by the following.
1) There is no need for the overexpression or administration of a corresponding neurotrophic factor.
2) The molecular size required for activation can be considerably reduced in comparison with full-length Trk. This enables high-level expression of molecules. Further, since the degree of freedom in designing a nucleic acid construct is considerably increased, it is possible, for example, to more easily achieve high-level expression, improvement in gene introduction efficiency, and the like.

### (Fusion polypeptide)

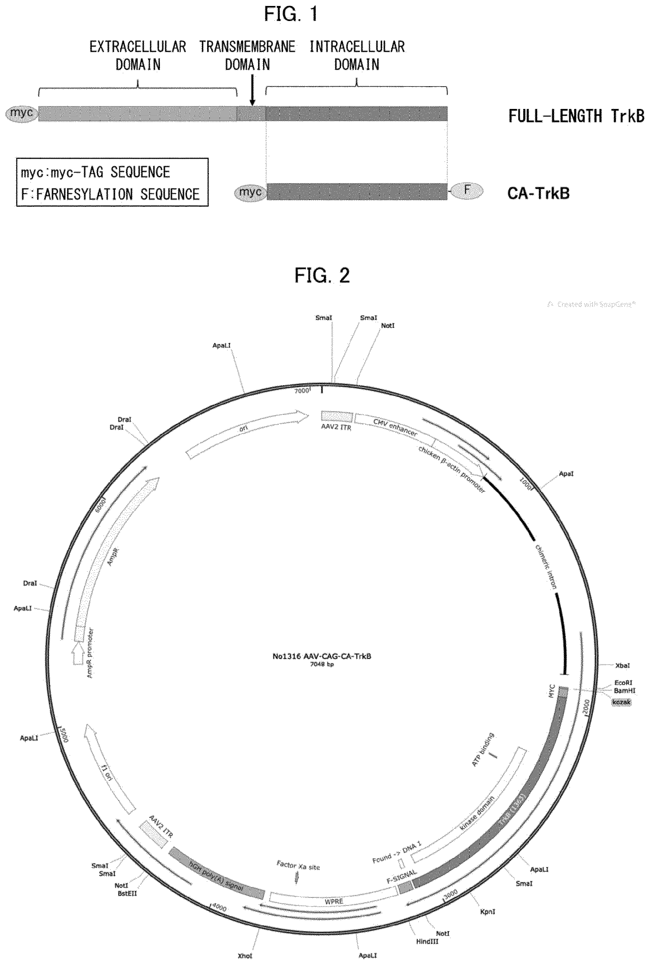

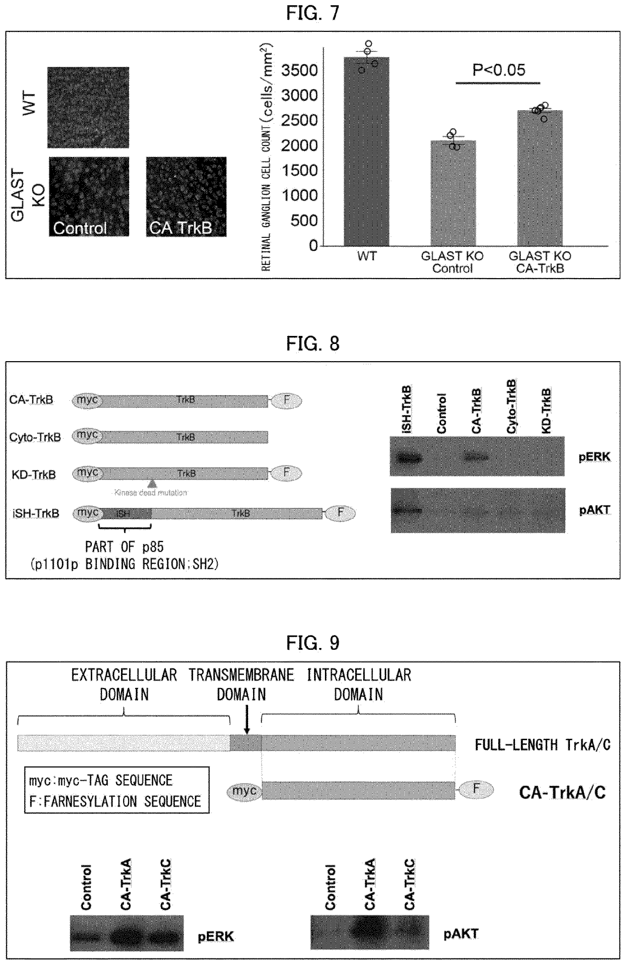

The fusion polypeptide encoded by the nucleic acid construct in accordance with the present embodiment includes (1) an intracellular domain of Trk and (2) a membrane localization sequence, each of which will be described later. It may be preferable that the membrane localization sequence be provided on the N-terminus side of the fusion polypeptide or on the C-terminus side of the fusion polypeptide, depending on the characteristics of the membrane localization sequence. In relation to the intracellular domain of Trk, the membrane localization sequence may be provided on the N-terminus side of the intracellular domain (on a side close to a transmembrane domain) or may be provided on the C-terminus side of the intracellular domain. Note that in constitutive active Trks shown in Examples, a membrane localization sequence is provided on a side that is the C-terminus side of a fusion polypeptide and is the C-terminus side of an intracellular domain of Trk. Thus, in each of the constitutive active Trks shown in the Examples, the intracellular domain of Trk is located on a side opposite to the side on which an intracellular domain is typically located, with respect to a cell membrane (see also Figs. 1 and 9).

In one example, the fusion polypeptide may include "another sequence" other than "the intracellular domain of Trk and the membrane localization sequence". Examples of the "another sequence" include a spacer sequence and an SH2 sequence of p85. The position in which the "another sequence" is inserted can be set according to the purpose of inserting the "another sequence". For example, the position can be between the intracellular domain of Trk and the membrane localization sequence, or on the N-terminus side of the fusion polypeptide (in this case, it may be preferable that the membrane localization sequence be provided on the C-terminus side), or on the C-terminus side of the fusion polypeptide (in this case, it may be preferable that the membrane localization sequence be provided on the N-terminus side).

In a preferable example, the number of amino acids in the fusion polypeptide is in a range of 300 to 650, and may be more preferably in a range of 330 to 630. In a preferable example, the number of amino acids in "the intracellular domain of Trk and the membrane localization sequence" in the fusion polypeptide is in a range of 300 to 450, and may be more preferably in a range of 330 to 440, 350 to 440, 360 (or 370) to 440, or 360 (or 370) to 410. The fusion polypeptide having a small size has advantages such as, for example, (1) an increased degree of freedom in designing other features in the nucleic acid construct (that is, features other than a region encoding the fusion polypeptide) and (2) an improved expression efficiency of the fusion polypeptide. In particular, having a wider range of options for a promoter and the like in the nucleic acid construct significantly contributes to improving the expression efficiency of the fusion polypeptide.

In one example, the fusion polypeptide may include a part of an extracellular domain of Trk and/or a part of a transmembrane domain. In a preferable example, the fusion polypeptide does not include the extracellular domain of Trk and/or the transmembrane domain. In a more preferable example, the fusion polypeptide includes neither the extracellular domain of Trk nor the transmembrane domain.

### (Intracellular domain of Trk)

In the present embodiment, the intracellular domain of Trk refers to (i) a polypeptide corresponding to an intracellular domain of a high-affinity receptor (Trk) for a neurotrophic factor, which high-affinity receptor is a single-span transmembrane protein, and (ii) a polypeptide functionally equivalent to this polypeptide. The intracellular domain is also referred to as a cytoplasmic domain.

Currently, the following three types of Trk family members have been reported: TrkA, TrkB, and TrkC. The extracellular domains of TrkA, TrkB, and TrkC are each a domain receiving a neurotrophic factor, and the intracellular domains of TrkA, TrkB, and TrkC each have tyrosine kinase activity. In a neural cell, TrkA, TrkB, and TrkC respectively receive corresponding neurotrophic factors. The reception of the neurotrophic factors triggers activation of respective intracellular signaling pathways downstream of TrkA, TrkB, and TrkC. Note that TrkA is a receptor for NGF, TrkB is a receptor for NT-4/5, and TrkC is a receptor for NT-3.

The intracellular domain of Trk is only required to (1) have tyrosine kinase activity and/or (2) be capable of activating an intracellular signaling pathway downstream of Trk in a case where the intracellular domain is localized at a cell membrane. The activation of the intracellular signaling pathway downstream of Trk can be recognized by a known technique, for example, by a technique of recognizing the activation in the form of an increased expression of at least one selected from Ras, ERK1/2, PI3K, Akt, phospholipase C-y, and the like.

Examples of the intracellular domain of Trk preferably include, but are not particularly limited to, the following.
1) A polypeptide having an amino acid sequence represented by any one of SEQ ID NO. 2, 6, 9, 12, or 14. Note that SEQ ID NO. 2 is an example of an amino acid sequence of an intracellular domain of human-derived TrkB, SEQ ID NO. 6 is an example of an amino acid sequence of an intracellular domain of mouse-derived TrkA, SEQ ID NO. 9 is an example of an amino acid sequence of an intracellular domain of mouse-derived TrkC, SEQ ID NO. 12 is an example of an amino acid sequence of an intracellular domain of human-derived TrkA, and SEQ ID NO. 14 is an example of an amino acid sequence of an intracellular domain of human-derived TrkC.
2) A polypeptide functionally equivalent to a polypeptide having an amino acid sequence represented by any one of SEQ ID NO. 2, 6, 9, 12, or 14. Examples of the functionally equivalent polypeptide include a polypeptide having an amino acid sequence having a sequence identity of 90% or more with respect to an amino acid sequence represented by any one of SEQ ID NO: 2, 6, or 9. The sequence identity is more preferably 91% or more, 92% or more, 93% or more, 94% or more, 95% or more, 96% or more, 97% or more, 98% or more, or 99% or more.
A 104th amino acid (Lys) in the amino acid sequence represented by SEQ ID NO: 6 is an amino acid sequence essential for tyrosine kinase activity or for activation of an intracellular signaling pathway downstream of TrkA, and thus preferably includes no mutation.
Similarly, a 134th amino acid (Lys) in the amino acid sequence represented by SEQ ID NO: 2 is an amino acid sequence essential for tyrosine kinase activity or for activation of an intracellular signaling pathway downstream of TrkB, and thus preferably includes no mutation.
Similarly, a 118th amino acid (Lys) in the amino acid sequence represented by SEQ ID NO: 9 is an amino acid sequence essential for tyrosine kinase activity or for activation of an intracellular signaling pathway downstream of TrkC, and thus preferably includes no mutation.
Similarly, a 104 amino acid (Lys) in the amino acid sequence represented by SEQ ID NO: 12 is an amino acid sequence essential for tyrosine kinase activity or for activation of an intracellular signaling pathway downstream of TrkA, and thus preferably includes no mutation.
Similarly, a 118th amino acid (Lys) in the amino acid sequence represented by SEQ ID NO: 14 is an amino acid sequence essential for tyrosine kinase activity or for activation of an intracellular signaling pathway downstream of TrkC, and thus preferably includes no mutation.

### (Membrane localization sequence)

The membrane localization sequence is a peptide sequence for causing the above fusion polypeptide to be localized at a cell membrane. The membrane localization sequence preferably induces lipid modification of an amino acid. The type of the lipid modification of an amino acid is not particularly limited, but can be, for example, (1) fatty-acylation, (2) prenylation, (3) glycosylphosphatidylinositolation, (4) cholesterolation, or the like, and more specifically myristoylation, palmitoylation, farnesylation, geranylgeranylation, or the like, and preferably farnesylation or geranylgeranylation.

Specific examples of an amino acid sequence motif constituting the membrane localization sequence include the following:
(1) Met-Gly-X-X-X-Ser-X-X-X (an N-terminus side lipid modification motif (N-myristoylation)); and (2) a CAAX motif, a CC motif, a CXC motif, a CCXX motif (a C-terminus side lipid modification motif) where X is any amino acid, A is an aliphatic amino acid (e.g., isoleucine, leucine, valine, alanine, methionine), and C is cysteine.

### (Aspect of nucleic acid construct)

An aspect of the nucleic acid construct in accordance with the present embodiment is a gene encoding the above fusion polypeptide including the intracellular domain of Trk and the membrane localization sequence. An example of the gene can be a gene having a base sequence represented by any one of SEQ ID NO: 4, 5, 8, or 11, or can be a gene having a base sequence having a sequence identity of 85% or more, 90% or more, 91% or more, 92% or more, 93% or more, 94% or more, 95% or more, 96% or more, 97% or more, 98% or more, or 99% or more with respect to a base sequence represented by any one of SEQ ID NO: 4, 5, 8, or 11. Note that an example of a gene encoding the intracellular domain of Trk included in the above fusion polypeptide can be a gene having a base sequence represented by any one of SEQ ID NO: 3, 7, 10, 13, or 15, or can be a gene having a base sequence having a sequence identity of 85% or more, 90% or more, 91% or more, 92% or more, 93% or more, 94% or more, 95% or more, 96% or more, 97% or more, 98% or more, or 99% or more with respect to a base sequence represented by any one of SEQ ID NO: 3, 7, 10, 13, or 15.

Another aspect of the nucleic acid construct in accordance with the present embodiment is an expression cassette which causes the above gene to be expressed. The expression cassette is preferably an expression vector, and more preferably a virus vector such as a retrovirus vector, a lentivirus vector, an adenovirus vector, an adeno-associated virus vector, a herpesvirus vector, and a vaccinia virus vector. Among these examples, from the viewpoint of use in a gene therapy against a nerve-related disease, the adeno-associated virus vector is particularly preferable. In particular, from the viewpoint of use in a gene therapy against a nerve-related disease, it may be preferable that the gene be an adeno-associated virus vector such as a serotype 1 (AAV1) having a tissue tropism for a central nervous system and the like, a serotype 2 (AAV2) having a broad tissue tropism, and a serotype 5 (AAV5) having a tissue tropism for a central nervous system, retina, and the like.

The above expression cassette includes any expression regulatory region that is functional in an expression host. A promoter serving as the expression regulatory region is not limited to a particular type, but specifically, for example, it may be preferable that the promoter be a promoter that enables high-level expression in a mammal (in particular, a human) or selectively functions in a specific tissue, such as a CAG promoter, a CMV promoter, and SYN (synapsin) I. Among these examples, the CAG promoter may be particularly preferable as the promoter. In the expression cassette, the promoter is provided on an upstream side of the gene.

The above expression cassette may further include, as necessary, a functional sequence that has an effect of, for example, increasing the stability of mRNA produced from transcription and serves to achieve an improvement of gene expression and the like. Examples of the functional sequence include a WPRE (woodchuck hepatitis virus posttranscriptional regulatory element) sequence. In the expression cassette, the WPRE sequence is provided on a downstream side of the gene.

Other examples of the functional sequence that can be included in the above expression cassette include a poly-A tail addition signal sequence. Examples of the poly-A tail addition signal sequence include a poly-A tail addition signal sequence of simian virus 40 and a poly-A tail addition signal sequence of human growth hormone. The poly-A tail addition signal sequence of human growth hormone may be more preferable. In the expression cassette, the WPRE sequence is provided on a downstream side of the gene.

The above expression cassette may further include inverted terminal repeat sequences (ITRs) and the like. ITRs are provided in the expression cassette so as to sandwich an entire transcription unit (from the promoter to the poly-A tail addition signal sequence) therebetween.

### [2. Pharmaceutical composition]

A pharmaceutical composition in accordance with an embodiment of the present invention is a gene therapeutic drug containing the above nucleic acid construct. In one example, the pharmaceutical composition is a drug for a nerve-related disease. In a more specific example, the pharmaceutical composition is for neuroprotection or neuroregeneration. The pharmaceutical composition exhibits a high neuroprotection effect and a high axon regeneration effect and can be used for treatment and/or prevention of a nerve-related disease. The scope of the present invention encompasses a method for treatment and/or prevention of a nerve-related disease, including administration of an effective amount of the pharmaceutical composition to an administration target.

### (Administration target)

A human or a non-human animal to be an administration target is preferably any one selected from the group consisting of mammals including a human. The type of a mammal to be an administration target is not particularly limited, but can be a laboratory animal such as a mouse, a rat, a rabbit, a guinea pig, and a primate other than a human; a pet animal such as a dog and a cat; a farm animal such as a cow and a horse; or a human, and particularly preferably a human. The human or the non-human animal to be an administration target has, for example, a nerve-related disease described later.

### (Nerve-related disease)

The nerve-related disease to be treated and/or prevented is not limited to a particular type, but refers to a pathological condition in which the function of a neural cell is impaired due to denaturation of the neural cell and/or cell death of the neural cell. The causes of the denaturation and cell death of the neural cell are not particularly limited and include a damage of the nervous system resulting from an accident or the like.

Examples of a nerve-related disease of an optic nerve system include: a neurodegenerative disease such as glaucoma, retinitis pigmentosa, age-related macular degeneration, and diabetic retinopathy; and a damage of an optic nerve system resulting from an accident or the like. In particular, a disease accompanied by degeneration of retinal ganglion cells typified by glaucoma and a damage of an optic nerve system (optic nerve injury) may be preferable as a disease to which the present invention is applied. Examples of a nerve-related disease of an auditory nervous system include: a sensory nerve degenerative disease such as nervous deafness; and a damage of an auditory nervous system resulting from an accident or the like.

### (Administration method, dosage, number of times of administration, and the like)

A method of administering the pharmaceutical composition is not particularly limited. The pharmaceutical composition may be administered topically by a method such as injection (with use of a syringe, an injection pump, or the like), instillation to an eye, transdermal administration, or sublingual administration, or may be administered systemically by a method such as oral administration, intravascular administration such as intravenous administration or intraarterial administration, or enteral administration. In a preferable aspect of administration, the pharmaceutical composition is administered topically to a part in the vicinity of a nervous system to be subjected to treatment or the like.

The dosage (effective amount) of the pharmaceutical composition may be set as appropriate according to the age and sex of the human or animal to be an administration target, the symptom, the administration route, the number of times of administration, and the like. As necessary, an *in vivo* assay with use of the pharmaceutical composition may be carried out in advance to determine the dosage without undue experiment.

The number of times of administration of the pharmaceutical composition is not particularly limited as long as an effect is obtained, and can be set as appropriate, for example, according to the dosage, the administration route, the symptom, the age and sex of the human or animal.

### (Component other than nucleic acid construct)

A pharmaceutical composition in accordance with an embodiment of the present invention can contain at least the above nucleic acid construct and a pharmaceutically-acceptable carrier. The pharmaceutically-acceptable carrier is not particularly limited, but is preferably one that does not substantially inhibit the function of the nucleic acid construct and does not have a substantial adverse effect on the human or non-human animal to be an administration target. It may be preferable that the pharmaceutical composition contain a liquid carrier such as water. In one example, the pharmaceutical composition may be a liposomal preparation.

Examples of the component constituting the above pharmaceutical composition further include, but are not particularly limited to, a lubricant, a preservative, a stabilizer, a wetting agent, an emulsifier, an osmotic pressure controlling salt, a buffer agent, a coloring agent, an antioxidant, and a viscosity modifier.

### [3. Cell activation method and the like]

A method for cell activation in accordance with an embodiment of the present invention is a method including the step (localization step) of causing an intracellular domain of Trk to be localized at a cell membrane via a membrane localization sequence in a cell. The localization step can typically be carried out by causing one selected from a nucleic acid construct described in the section [1. Nucleic acid construct] and a pharmaceutical composition described in the section [2. Pharmaceutical composition] to be expressed in a neural cell. The type of the neural cell is not particularly limited, and examples of the neural cell include a cell of a central nervous system, a cell of a peripheral nervous system, and a precursor cell of any of these cells. The neural cell may be an isolated neural cell, or may be a neural cell present in a body of a human or a non-human animal.

According to the method for cell activation, it is possible to activate an intracellular signaling pathway downstream of Trk without administration of a neurotrophic factor. As a result of the cell activation, a high neuroprotection effect and a high axon regeneration effect can be exhibited. Thus, the method for cell activation can be used for treatment and/or prevention of a nerve-related disease.

An embodiment of the present invention further provides a neural cell into which a nucleic acid construct described in the section [1. Nucleic acid construct] has been introduced in an expressible manner, a non-human animal having the neural cell, and the like.

### [4. Recap]

As described above, the present invention encompasses the following aspects.
(1) A nucleic acid construct encoding a fusion polypeptide, the fusion polypeptide including: an intracellular domain of Trk; and a membrane localization sequence.
(2) The nucleic acid construct as set forth in (1), wherein the intracellular domain of Trk is one selected from:
   1) a polypeptide having an amino acid sequence represented by any one of SEQ ID NO. 2, 6, 9, 12, or 14; and
   2) a polypeptide having an amino acid sequence having a sequence identity of 90% or more with respect to an amino acid sequence represented by any one of SEQ ID NO. 2, 6, 9, 12, or 14.
(3) The nucleic acid construct as set forth in (1) or (2), wherein the membrane localization sequence induces lipid modification of an amino acid.
(4) The nucleic acid construct as set forth in (3), wherein the membrane localization sequence is a farnesylation sequence.
(5) The nucleic acid construct as set forth in any one of (1) to (4), being a vector.
(6) The nucleic acid construct as set forth in (5), being an adeno-associated virus vector.
(7) A pharmaceutical composition containing a nucleic acid construct recited in any one of (1) to (6).
(8) The pharmaceutical composition as set forth in (7), being a drug for a nerve-related disease.
(9) The pharmaceutical composition as set forth in (8), being for neuroprotection or neuroregeneration.
(10) A neural cell into which a nucleic acid construct recited in (1) has been introduced in an expressible manner.
(11) A method for cell activation, including the step of causing an intracellular domain of Trk to be localized at a cell membrane via a membrane localization sequence in a cell.
(12) The method as set forth in (11), wherein the step is carried out by causing one selected from a nucleic acid construct recited in any one of (1) to (6) and a pharmaceutical composition recited in any one of (7) to (9) to be expressed in a neural cell.
(13) A fusion polypeptide including: an intracellular domain of Trk; and a membrane localization sequence. The fusion polypeptide may be encoded by a nucleic acid construct recited in any one of (1) to (4).

The following description will more specifically discuss an embodiment of the present invention with reference to Examples. It is a matter of course that the present invention is not limited to the Examples below and that details of the present invention can have various aspects. Further, the present invention is not limited to the embodiments, but can be altered by a skilled person in the art within the scope of the claims. The present invention also encompasses, in its technical scope, any embodiment derived by combining technical means disclosed in differing embodiments. All literatures described in this specification are incorporated herein by reference.

### Examples

### [Example 1] Preparation of constitutive active TrkB (hereinafter, CA-TrkB) vector

### (Method)

An AAV vector (referred to as "AAV-CA-TrkB") expressing an intracellular domain of a TrkB molecule was prepared in the following manner. As an AAV vector in which to incorporate a gene fragment encoding CA-TrkB, used was pAAV-CAG-shuttle-WPRE manufactured by Applied Viromics. A DNA fragment encoding a protein having a myc-tag added to the N-terminus of an intracellular domain of TrkB and a farnesylation signal sequence added to the C-terminus of the intracellular domain was prepared as a gene fragment encoding CA-TrkB (see also Fig. 1). That is, CA-TrkB was provided by: using only the intracellular domain of TrkB without including neither an extracellular domain of TrkB nor a transmembrane domain; and adding the myc-tag and the farnesylation signal sequence to the intracellular domain.

The farnesylation signal sequence (having a base sequence represented by SEQ ID NO: 1) is a signal sequence for causing a protein to be localized at a membrane. The amino acid sequence of the intracellular domain of TrkB is represented by SEQ ID NO: 2, and the base sequence of a gene (DNA) encoding the intracellular domain of TrkB is represented by SEQ ID NO: 3. The base sequence of the gene fragment (the full length of the gene fragment inserted into the AAV vector) encoding CA-TrkB is represented by SEQ ID NO: 4.

AAV-CA-TrkB was designed with an aim to achieve high-level expression, by using a CAG promoter as an expression regulatory system for the gene fragment encoding CA-TrkB and further fusing a WPRE sequence and a poly-A sequence derived from human growth hormone together (see Fig. 2). The WPRE sequence and the poly-A sequence derived from human growth hormone were provided downstream of the farnesylation signal sequence in the AAV vector. The CAG promoter, the WPRE sequence, and the poly-A sequence derived from human growth hormone had been incorporated in the original AAV vector from the start.

Subsequently, the obtained AAV-CA-TrkB was transfected into Cos7 cells, and evaluation of the expression level of AAV-CA-TrkB and activity was carried out.

### (Results)

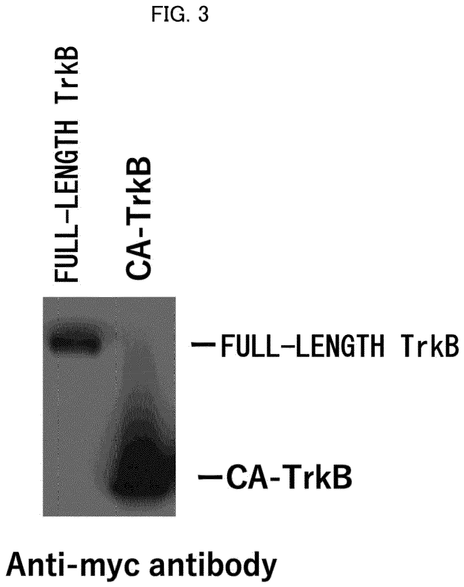

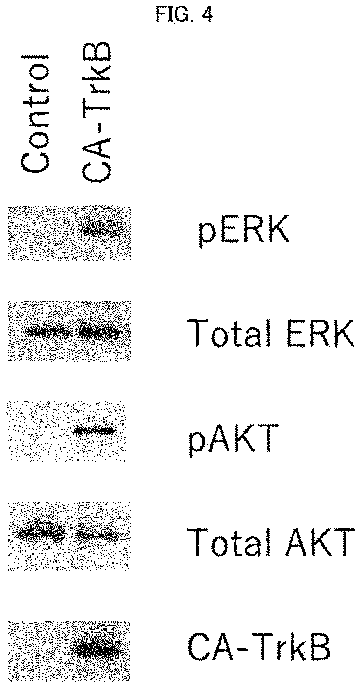

The results of transfecting AAV-CA-TrkB into the Cos7 cells are shown in Figs. 3 and 4. Fig. 3 shows results obtained from transfecting AAV-CA-TrkB into the Cos7 cells, culturing the cells for 24 hours, and then dissolving the cells and conducting Western blotting with use of an anti-myc antibody (c-Myc antibody (9E10) manufactured by Santa Cruz). It was found that AAV-CA-TrkB had a clearly increased expression level in comparison with a full-length TrkB vector which was subjected to a similar experiment. Note that the full-length TrkB vector is an adeno-associated virus vector in which, in contrast to the structure of AAV-CA-TrkB, a gene fragment encoding full-length TrkB is inserted instead of a gene fragment encoding CA-TrkB.

Fig. 4 shows results obtained from transfecting AAV-CA-TrkB into Cos7 cells, culturing the cells for 24 hours, and then dissolving the cells and conducting Western blotting with use of an anti-pERK antibody (Phospho-p44/42 (Erk1/2) (Thr202/Thr204) manufactured by Cell Signaling Technology), an anti-pAKT antibody (Phospho-Akt (Ser473) (587F11) manufactured by Cell Signaling Technology), an anti-ERK antibody (p44/42 MAPK (Erk1/2) (137F5) manufactured by Cell Signaling Technology), and an anti-AKT antibody (Akt antibody manufactured by Cell Signaling Technology). In the Cos7 cells in which AAV-CA-TrkB was expressed, increased production levels of pERK and pAKT were observed, and it was thus indicated that both signals were activated. However, no changes were observed in total amounts of ERK and AKT. Note that pERK and pAKT are intracellular signals provided downstream of TrkB. A control in Fig. 4 indicates results of a similar experiment conducted with use of an empty vector (a vector obtained by removing the CA-TrkB sequence from the AAV-CA-TrkB vector), and the results show that pERK and pAKT were hardly produced.

### [Example 2] Pathological analysis of optic nerve-crushed model

### (Method)

### - Evaluation method of neuroprotection effect -

To mice (line name: C57BL/6, obtained from Charles River), 2 µL of AAV-DsRed (control group) or AAV-CA-TrkB was administered into an eyeball 14 days before crushing an optic nerve. AAV-DsRed is an adeno-associated virus vector in which, in contrast to the structure of AAV-CA-TrkB, a gene fragment encoding fluorescent protein DsRed is inserted instead of a gene fragment encoding CA-TrkB.

Subsequently, an optic nerve of each of the mice was exposed, and the optic nerve was physically crushed in a portion approximately 1 mm from the backside of the eyeball (see also Non-patent Literatures 6 and 7 for details of the method). Then, 14 days after the crushing of the optic nerve, an expanded specimen of a retina was prepared, and the number of remaining retinal ganglion cells was quantitatively counted. Note that 10 days before the optic nerve was collected (i.e., 10 days before the expanded specimen of the retina was prepared), a dye Fluoro-Gold (the product name of a product manufactured by Fluorochrome) had been injected from the superior colliculus of the brain to retrograde label retinal ganglion cells.

### - Evaluation method of optic nerve regeneration -

To mice (line name: C57BL/6, obtained from Charles River), 2 µL of AAV-DsRed (control group) or AAV-CA-TrkB was administered into an eyeball 14 days before crushing an optic nerve.

Crushing of the optic nerve was carried out similarly as discussed in "Evaluation method of neuroprotection effect" above. Then, 14 days after the crushing of the optic nerve, each mouse was subjected to perfusion fixation, a pathological specimen of the optic nerve was prepared, and the amount of axon regeneration from the part at which the optic nerve had been crushed was quantified. Note that 3 days before the optic nerve was collected (i.e., 3 days before the pathological specimen of the optic nerve was prepared), a fluorescent dye CTB had been injected from the eyeball to color regenerated axonal fibers.

### (Results)

### - Neuroprotection effect -

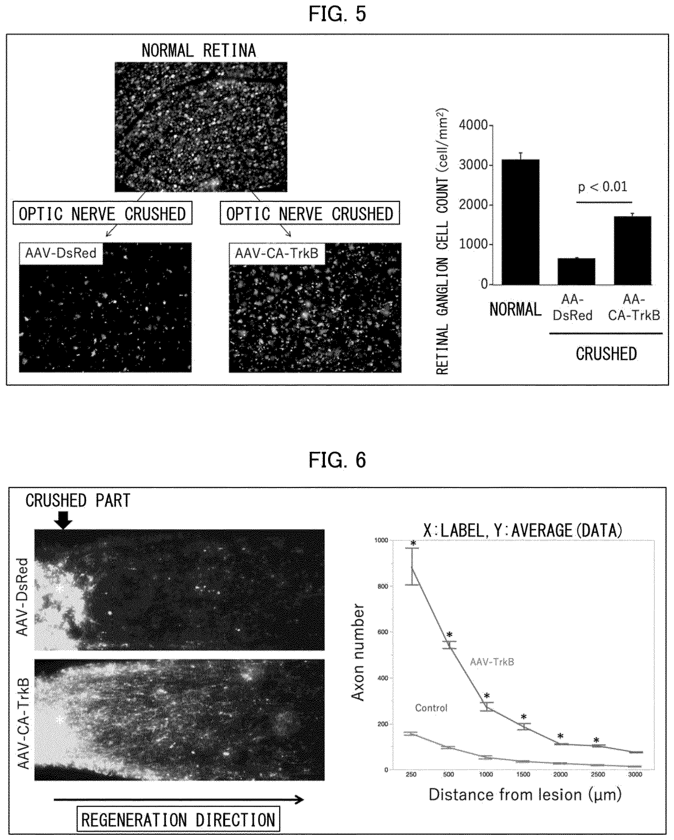

Fourteen days after the crushing of the optic nerve, the number of remaining retinal ganglion cells was quantified. A significant increase was observed in the number of remaining retinal ganglion cells in the group to which AAV-CA-TrkB had been administered (1720 ± 72 cells/mm²), as compared with the group to which AAV-DsRed had been administered (664 ± 14 cells/mm²) (Fig. 5). Note that p<0.01 (one-tailed Student's t test).

### - Optic nerve regeneration -

Fourteen days after the optic nerve was crushed, the regenerated axons labeled with CTB were measured. The measurement indicated that the group to which AAV-CA-TrkB had been administered had a significant improvement both in the number of regenerated axons and the length of axonal outgrowth, as compared with the group to which AAV-DsRed had been administered (Fig. 6). The X-axis and the Y-axis of the graph in Fig. 6 respectively indicate a distance (pm) from the part at which the optic nerve had been crushed and the number of axons. Note that p<0.05 (one-tailed Student's t test).

### [Example 3] Retinal ganglion cell protection effect by CA-TrkB (glaucoma model mice)

### (Method)

A GLAST knockout mouse, which is a glaucoma model mouse (Reference research paper: Harada T, et al. The potential role of glutamate transporters in the pathogenesis of normal tension glaucoma. The Journal of Clinical Investigation 117: 1763-1770, 2007.), loses retinal ganglion cells to experience decreased visual performance at the age of 3 weeks to 5 weeks. To 10-week-old GLAST knockout mice, 1 µL of AAV-DsRed (control group) or AAV-CA-TrkB was administered into an eyeball (see also Example 2). Then, an expanded specimen of a retina of these knockout mice was prepared when the mice had become 12 weeks old, and the number of remaining retinal ganglion cells was quantitatively counted. To detect the retinal ganglion cells, immunostaining using an RBPMS antibody (an anti-RBPMS antibody manufactured by Merck Millipore) was employed. RBPMS is a marker for retinal ganglion cells.

### (Results)

As shown in Fig. 7, a significant increase was observed in the number of remaining retinal ganglion cells in the group to which AAV-CA-TrkB had been administered, as compared with the control group. Note that p<0.05 (one-tailed Student's t test).

### [Example 4]

### (Method)

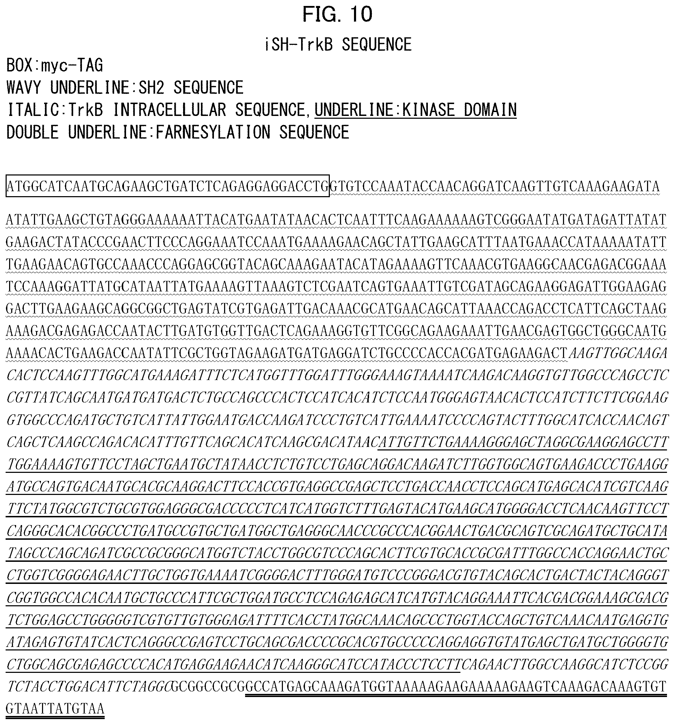

Next, the following vectors expressing a mutant (see also Fig. 8) of CA-TrkB were used to examine signaling activity.
- A "Cyto-TrkB" vector: a vector obtained by incorporating, into the AAV vector of Example 1, a DNA fragment encoding a protein including an intracellular domain of TrkB and a myc-tag added to the N-terminus side of the intracellular domain. The "Cyto-TrkB" vector differs from AAV-CA-TrkB of Example 1 only in that no farnesylation signal sequence is added to the C-terminus side of the intracellular domain of TrkB.
- A "KD-TrkB" vector: differing from AAV-CA-TrkB of Example 1 only in that an encoded protein includes, in an intracellular domain of TrkB, one amino acid mutation (a lysine to asparagine mutation at the 134th amino acid) that causes a loss of the kinase activity of the intracellular domain.
- An "iSH-TrkB" vector: differing from AAV-CA-TrkB of Example 1 only in that an encoded protein further includes an SH2 sequence of p85 inserted between the N-terminus of an intracellular domain of TrkB and a myc-tag. Note that the SH2 sequence is a sequence necessary for forming a complex with a constituent factor p110 of PI3K. The base sequence of a gene fragment (the full length of a gene fragment inserted into the AAV vector) encoding iSH-TrkB is represented by SEQ ID NO: 5 and illustrated as Fig. 10.

Subsequently, in a similar manner to Example 1, these vectors were transfected into Cos7 cells, the cells were dissolved after 24 hours of culturing, and Western blotting was conducted to examine effect on activation of ERK and AKT.

### (Results)

As shown in Fig. 8, in the cases where the "Cyto-TrkB" vector and the "KD-TrkB" vector were transfected, no increases of pERK and pAKT were observed, and no activations of pERK signaling and pAKT signaling were thus indicated. In contrast, in the case where the "iSH-TrkB" vector was transfected, a significant increase in AKT activity was indicated in comparison with the case where AAV-CA-TrkB was transfected (in Fig. 8, indicated as "CA-TrkB"). Note that a control in Fig. 8 is the same as the control in Example 1.

### [Example 5] Preparation of constitutive active TrkA vector and constitutive active TrkC vector

### (Method)

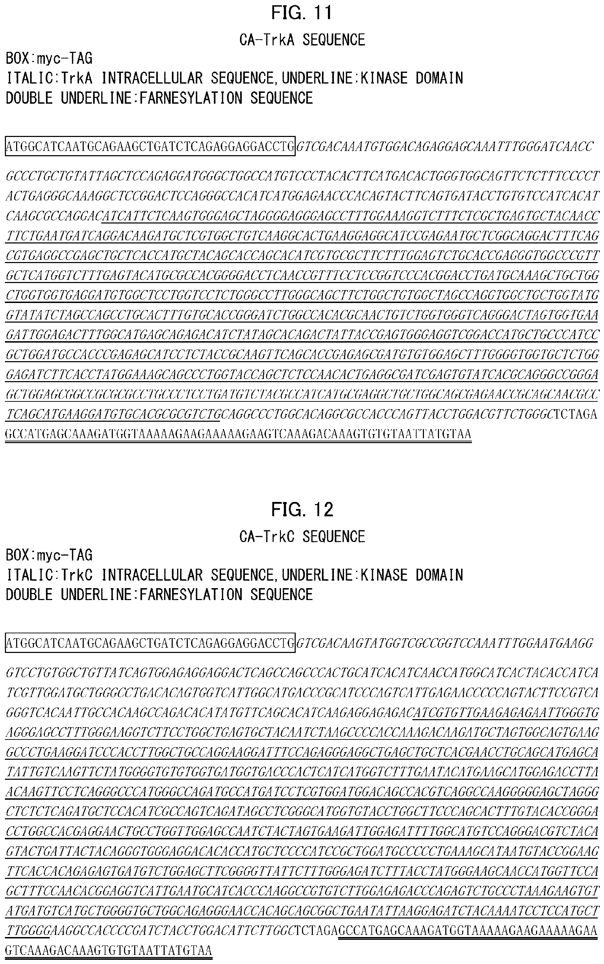

An AAV vector (referred to as "AAV-CA-TrkA") expressing an intracellular domain of a TrkA molecule was prepared in accordance with the method of Example 1. That is, AAV-CA-TrkA was prepared in accordance with the method described in Example 1 with use of a fragment having the same structure as the gene fragment encoding CA-TrkB (see Example 1) except that an intracellular domain of TrkA is encoded instead of an intracellular domain of TrkB (see also Fig. 9).
The amino acid sequence of the intracellular domain of TrkA is represented by SEQ ID NO: 6, and the base sequence of a gene (DNA) encoding the intracellular domain of TrkA is represented by SEQ ID NO: 7. The base sequence of the gene fragment (the full length of the gene fragment inserted into the AAV vector) encoding CA-TrkA is represented by SEQ ID NO: 8 and illustrated as Fig. 11.

Similarly, an AAV vector (referred to as "AAV-CA-TrkC") expressing an intracellular domain of a TrkC molecule was prepared in accordance with the method of Example 1. That is, AAV-CA-TrkC was prepared in accordance with the method described in Example 1 with use of a fragment having the same structure as the gene fragment encoding CA-TrkB (see Example 1) except that an intracellular domain of TrkC is encoded instead of an intracellular domain of TrkB (see also Fig. 9).
The amino acid sequence of the intracellular domain of TrkC is represented by SEQ ID NO: 9, and the base sequence of a gene (DNA) encoding the intracellular domain of TrkC is represented by SEQ ID NO: 10. The base sequence of the gene fragment (the full length of the gene fragment inserted into the AAV vector) encoding CA-TrkC is represented by SEQ ID NO: 11 and illustrated as Fig. 12.

Subsequently, in a similar manner to Example 1, these vectors were transfected into Cos7 cells, the cells were dissolved after 24 hours of culturing, and Western blotting was conducted to examine effect of TrkA and TrkC, which are homologues of TrkB, on activation of ERK and AKT.

### (Results)

As shown in Fig. 9, in the cases where AAV-CA-TrkA and AAV-CA-TrkC were transfected, increases of pERK and pAKT were observed, and it was thus indicated that both pERK signaling and pAKT signaling were activated. Note that a control in Fig. 9 is the same as the control in Example 1.

### [Example 6] Visual performance recovery effect by CA-TrkB (optic nerve-crushed model mice)

### (Method)

From mature mice, a group to which AAV-CA-TrkB was administered was obtained in accordance with the method in Example 2. Fourteen days later, an optic nerve was crushed. Fourteen days and 28 days after the crushing, an electrode port was operatively attached to the superior colliculus of the brain, and a flash-visual evoked potential (F-VEP) was measured.

### (Results)

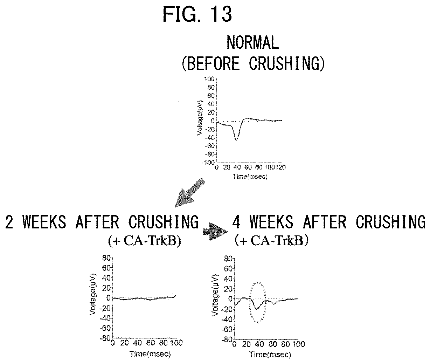

As shown in Fig. 13, reaction of the visual evoked potential was lost 2 weeks after the crushing, but generation of the visual evoked potential was observed 4 weeks after the crushing. It was thus confirmed that the visual performance had partially recovered.

### Industrial Applicability

The present invention can be used for medical purposes involving, for example, nerve-related diseases and the like.

## Claims

1. A nucleic acid construct encoding a fusion polypeptide, the fusion polypeptide including: an intracellular domain of Trk; and a membrane localization sequence.

2. The nucleic acid construct as set forth in claim 1, wherein the intracellular domain of Trk is one selected from:
1) a polypeptide having an amino acid sequence represented by any one of SEQ ID NO. 2, 6, 9, 12, or 14; and
2) a polypeptide having an amino acid sequence having a sequence identity of 90% or more with respect to an amino acid sequence represented by any one of SEQ ID NO. 2, 6, 9, 12, or 14.

3. The nucleic acid construct as set forth in claim 1 or 2, wherein the membrane localization sequence induces lipid modification of an amino acid.

4. The nucleic acid construct as set forth in claim 3, wherein the membrane localization sequence is a farnesylation sequence.

5. The nucleic acid construct as set forth in any one of claims 1 to 4, being a vector.

6. The nucleic acid construct as set forth in claim 5, being an adeno-associated virus vector.

7. A pharmaceutical composition comprising a nucleic acid construct recited in any one of claims 1 to 6.

8. The pharmaceutical composition as set forth in claim 7, being a drug for a nerve-related disease.

9. The pharmaceutical composition as set forth in claim 8, being for neuroprotection or neuroregeneration.

10. A neural cell into which a nucleic acid construct recited in claim 1 has been introduced in an expressible manner.

11. A method for cell activation, comprising the step of causing an intracellular domain of Trk to be localized at a cell membrane via a membrane localization sequence in a cell.

12. The method as set forth in claim 11, wherein the step is carried out by causing one selected from a nucleic acid construct recited in any one of claims 1 to 6 and a pharmaceutical composition recited in any one of claims 7 to 9 to be expressed in a neural cell.

13. A fusion polypeptide comprising: an intracellular domain of Trk; and a membrane localization sequence.


SEQUENCE LISTING

<110>  Tokyo Metropolitan Institute of Medical Science

<120>  A neucleic acid construct coding for a Trk fragment, and use
       thereof

<130>  TIK20036

<150>  JP 2019-038503
<151>  2019-03-04

<160>  15

<170>  PatentIn version 3.5

<210>  1
<211>  63
<212>  DNA
<213>  Artificial Sequence

<220>
<223>  signal sequence

<400>  1
gccatgagca aagatggtaa aaagaagaaa aagaagtcaa agacaaagtg tgtaattatg      60

taa                                                                    63


<210>  2
<211>  384
<212>  PRT
<213>  Homo sapiens

<400>  2

Lys Leu Ala Arg His Ser Lys Phe Gly Met Lys Asp Phe Ser Trp Phe
1               5                   10                  15


Gly Phe Gly Lys Val Lys Ser Arg Gln Gly Val Gly Pro Ala Ser Val
            20                  25                  30


Ile Ser Asn Asp Asp Asp Ser Ala Ser Pro Leu His His Ile Ser Asn
        35                  40                  45


Gly Ser Asn Thr Pro Ser Ser Ser Glu Gly Gly Pro Asp Ala Val Ile
    50                  55                  60


Ile Gly Met Thr Lys Ile Pro Val Ile Glu Asn Pro Gln Tyr Phe Gly
65                  70                  75                  80


Ile Thr Asn Ser Gln Leu Lys Pro Asp Thr Phe Val Gln His Ile Lys
                85                  90                  95


Arg His Asn Ile Val Leu Lys Arg Glu Leu Gly Glu Gly Ala Phe Gly
            100                 105                 110

Lys Val Phe Leu Ala Glu Cys Tyr Asn Leu Cys Pro Glu Gln Asp Lys
        115                 120                 125

Ile Leu Val Ala Val Lys Thr Leu Lys Asp Ala Ser Asp Asn Ala Arg
    130                 135                 140

Lys Asp Phe His Arg Glu Ala Glu Leu Leu Thr Asn Leu Gln His Glu
145                 150                 155                 160

His Ile Val Lys Phe Tyr Gly Val Cys Val Glu Gly Asp Pro Leu Ile
                165                 170                 175

Met Val Phe Glu Tyr Met Lys His Gly Asp Leu Asn Lys Phe Leu Arg
            180                 185                 190

Ala His Gly Pro Asp Ala Val Leu Met Ala Glu Gly Asn Pro Pro Thr
        195                 200                 205

Glu Leu Thr Gln Ser Gln Met Leu His Ile Ala Gln Gln Ile Ala Ala
    210                 215                 220

Gly Met Val Tyr Leu Ala Ser Gln His Phe Val His Arg Asp Leu Ala
225                 230                 235                 240

Thr Arg Asn Cys Leu Val Gly Glu Asn Leu Leu Val Lys Ile Gly Asp
                245                 250                 255

Phe Gly Met Ser Arg Asp Val Tyr Ser Thr Asp Tyr Tyr Arg Val Gly
            260                 265                 270

Gly His Thr Met Leu Pro Ile Arg Trp Met Pro Pro Glu Ser Ile Met
        275                 280                 285

Tyr Arg Lys Phe Thr Thr Glu Ser Asp Val Trp Ser Leu Gly Val Val
    290                 295                 300

Leu Trp Glu Ile Phe Thr Tyr Gly Lys Gln Pro Trp Tyr Gln Leu Ser
305                 310                 315                 320

Asn Asn Glu Val Ile Glu Cys Ile Thr Gln Gly Arg Val Leu Gln Arg
                325                 330                 335

Pro Arg Thr Cys Pro Gln Glu Val Tyr Glu Leu Met Leu Gly Cys Trp
            340                 345                 350

Gln Arg Glu Pro His Met Arg Lys Asn Ile Lys Gly Ile His Thr Leu

355                 360                 365

Leu Gln Asn Leu Ala Lys Ala Ser Pro Val Tyr Leu Asp Ile Leu Gly
    370                 375                 380

<210>  3
<211>  1152
<212>  DNA
<213>  Homo sapiens

<400>  3
aagttggcaa gacactccaa gtttggcatg aaagatttct catggtttgg atttgggaaa      60

gtaaaatcaa gacaaggtgt tggcccagcc tccgttatca gcaatgatga tgactctgcc     120

agcccactcc atcacatctc caatgggagt aacactccat cttcttcgga aggtggccca     180

gatgctgtca ttattggaat gaccaagatc cctgtcattg aaaatcccca gtactttggc     240

atcaccaaca gtcagctcaa gccagacaca tttgttcagc acatcaagcg acataacatt     300

gttctgaaaa gggagctagg cgaaggagcc tttggaaaag tgttcctagc tgaatgctat     360

aacctctgtc ctgagcagga caagatcttg gtggcagtga agaccctgaa ggatgccagt     420

gacaatgcac gcaaggactt ccaccgtgag gccgagctcc tgaccaacct ccagcatgag     480

cacatcgtca agttctatgg cgtctgcgtg gagggcgacc ccctcatcat ggtctttgag     540

tacatgaagc atggggacct caacaagttc ctcagggcac acggccctga tgccgtgctg     600

atggctgagg gcaacccgcc cacggaactg acgcagtcgc agatgctgca tatagcccag     660

cagatcgccg cgggcatggt ctacctggcg tcccagcact tcgtgcaccg cgatttggcc     720

accaggaact gcctggtcgg ggagaacttg ctggtgaaaa tcggggactt tgggatgtcc     780

cgggacgtgt acagcactga ctactacagg gtcggtggcc acacaatgct gcccattcgc     840

tggatgcctc cagagagcat catgtacagg aaattcacga cggaaagcga cgtctggagc     900

ctgggggtcg tgttgtggga gattttcacc tatggcaaac agccctggta ccagctgtca     960

aacaatgagg tgatagagtg tatcactcag ggccgagtcc tgcagcgacc ccgcacgtgc    1020

ccccaggagg tgtatgagct gatgctgggg tgctggcagc gagagcccca catgaggaag    1080

aacatcaagg gcatccatac cctccttcag aacttggcca aggcatctcc ggtctacctg    1140

gacattctag gc                                                        1152

<210>  4
<211>  1263
<212>  DNA
<213>  Artificial Sequence

<220>
<223>  nucleic acid sequence coding for CA-TrkB

<400>  4

atggcatcaa tgcagaagct gatctcagag gaggacctga agttggcaag acactccaag 60

tttggcatga aagatttctc atggtttgga tttgggaaag taaaatcaag acaaggtgtt 120

ggcccagcct ccgttatcag caatgatgat gactctgcca gcccactcca tcacatctcc 180

aatgggagta acactccatc ttcttcggaa ggtggcccag atgctgtcat tattggaatg 240

accaagatcc ctgtcattga aaatccccag tactttggca tcaccaacag tcagctcaag 300

ccagacacat ttgttcagca catcaagcga cataacattg ttctgaaaag ggagctaggc 360

gaaggagcct ttggaaaagt gttcctagct gaatgctata acctctgtcc tgagcaggac 420

aagatcttgg tggcagtgaa gaccctgaag gatgccagtg acaatgcacg caaggacttc 480

caccgtgagg ccgagctcct gaccaacctc cagcatgagc acatcgtcaa gttctatggc 540

gtctgcgtgg agggcgaccc cctcatcatg gtctttgagt acatgaagca tggggacctc 600

aacaagttcc tcagggcaca cggccctgat gccgtgctga tggctgaggg caacccgccc 660

acggaactga cgcagtcgca gatgctgcat atagcccagc agatcgccgc gggcatggtc 720

tacctggcgt cccagcactt cgtgcaccgc gatttggcca ccaggaactg cctggtcggg 780

gagaacttgc tggtgaaaat cggggacttt gggatgtccc gggacgtgta cagcactgac 840

tactacaggg tcggtggcca cacaatgctg cccattcgct ggatgcctcc agagagcatc 900

atgtacagga aattcacgac ggaaagcgac gtctggagcc tgggggtcgt gttgtgggag 960

attttcacct atggcaaaca gccctggtac cagctgtcaa acaatgaggt gatagagtgt 1020

atcactcagg gccgagtcct gcagcgaccc cgcacgtgcc cccaggaggt gtatgagctg 1080

atgctggggt gctggcagcg agagccccac atgaggaaga acatcaaggg catccatacc 1140

ctccttcaga acttggccaa ggcatctccg gtctacctgg acattctagg cgcggccgcg 1200

gccatgagca aagatggtaa aaagaagaaa aagaagtcaa agacaaagtg tgtaattatg 1260

taa 1263

<210> 5
<211> 1851
<212> DNA
<213> Artificial Sequence

<220>
<223> nucleic acid sequence coding for iSH-TrkB

<400> 5
atggcatcaa tgcagaagct gatctcagag gaggacctgg tgtccaaata ccaacaggat 60

caagttgtca aagaagataa tattgaagct gtagggaaaa aattacatga atataacact 120

caatttcaag aaaaaagtcg ggaatatgat agattatatg aagactatac ccgaacttcc 180

caggaaatcc aaatgaaaag aacagctatt gaagcattta atgaaaccat aaaaatattt 240

gaagaacagt gccaaaccca ggagcggtac agcaaagaat acatagaaaa gttcaaacgt 300

gaaggcaacg agacggaaat ccaaaggatt atgcataatt atgaaaagtt aaagtctcga 360

atcagtgaaa ttgtcgatag cagaaggaga ttggaagagg acttgaagaa gcaggcggct 420

gagtatcgtg agattgacaa acgcatgaac agcattaaac cagacctcat tcagctaaga 480

aagacgagag accaatactt gatgtggttg actcagaaag gtgttcggca gaagaaattg 540

aacgagtggc tgggcaatga aaacactgaa gaccaatatt cgctggtaga agatgatgag 600

gatctgcccc accacgatga gaagactaag ttggcaagac actccaagtt tggcatgaaa 660

gatttctcat ggtttggatt tgggaaagta aaatcaagac aaggtgttgg cccagcctcc 720

gttatcagca atgatgatga ctctgccagc ccactccatc acatctccaa tgggagtaac 780

actccatctt cttcggaagg tggcccagat gctgtcatta ttggaatgac caagatccct 840

gtcattgaaa atccccagta ctttggcatc accaacagtc agctcaagcc agacacattt 900

gttcagcaca tcaagcgaca taacattgtt ctgaaaaggg agctaggcga aggagccttt 960

ggaaaagtgt tcctagctga atgctataac ctctgtcctg agcaggacaa gatcttggtg 1020

gcagtgaaga ccctgaagga tgccagtgac aatgcacgca aggacttcca ccgtgaggcc 1080

gagctcctga ccaacctcca gcatgagcac atcgtcaagt tctatggcgt ctgcgtggag 1140

ggcgaccccc tcatcatggt ctttgagtac atgaagcatg gggacctcaa caagttcctc 1200

agggcacacg gccctgatgc cgtgctgatg gctgagggca acccgcccac ggaactgacg 1260

cagtcgcaga tgctgcatat agcccagcag atcgccgcgg gcatggtcta cctggcgtcc 1320

cagcacttcg tgcaccgcga tttggccacc aggaactgcc tggtcgggga gaacttgctg 1380

gtgaaaatcg gggactttgg gatgtcccgg gacgtgtaca gcactgacta ctacagggtc 1440

ggtggccaca caatgctgcc cattcgctgg atgcctccag agagcatcat gtacaggaaa 1500

ttcacgacgg aaagcgacgt ctggagcctg ggggtcgtgt tgtgggagat tttcacctat 1560

ggcaaacagc cctggtacca gctgtcaaac aatgaggtga tagagtgtat cactcagggc 1620

cgagtcctgc agcgaccccg cacgtgcccc caggaggtgt atgagctgat gctggggtgc 1680

tggcagcgag agccccacat gaggaagaac atcaagggca tccataccct ccttcagaac 1740

ttggccaagg catctccggt ctacctggac attctaggcg cggccgcggc catgagcaaa 1800

gatggtaaaa agaagaaaaa gaagtcaaag acaaagtgtg taattatgta a 1851

<210> 6
<211> 356
<212> PRT
<213> Mus musculus

<400> 6

Lys Cys Gly Gln Arg Ser Lys Phe Gly Ile Asn Arg Pro Ala Val Leu
1               5                   10                  15

Ala Pro Glu Asp Gly Leu Ala Met Ser Leu His Phe Met Thr Leu Gly
            20                  25                  30

Gly Ser Ser Leu Ser Pro Thr Glu Gly Lys Gly Ser Gly Leu Gln Gly
        35                  40                  45

His Ile Met Glu Asn Pro Gln Tyr Phe Ser Asp Thr Cys Val His His
    50                  55                  60

Ile Lys Arg Gln Asp Ile Ile Leu Lys Trp Glu Leu Gly Glu Gly Ala
65                  70                  75                  80

Phe Gly Lys Val Phe Leu Ala Glu Cys Tyr Asn Leu Leu Asn Asp Gln
                85                  90                  95

Asp Lys Met Leu Val Ala Val Lys Ala Leu Lys Glu Ala Ser Glu Asn
            100                 105                 110

Ala Arg Gln Asp Phe Gln Arg Glu Ala Glu Leu Leu Thr Met Leu Gln
        115                 120                 125

His Gln His Ile Val Arg Phe Phe Gly Val Cys Thr Glu Gly Gly Pro
    130                 135                 140

Leu Leu Met Val Phe Glu Tyr Met Arg His Gly Asp Leu Asn Arg Phe
145                 150                 155                 160

Leu Arg Ser His Gly Pro Asp Ala Lys Leu Leu Ala Gly Gly Glu Asp
                165                 170                 175

Val Ala Pro Gly Pro Leu Gly Leu Gly Gln Leu Leu Ala Val Ala Ser
            180                 185                 190

Gln Val Ala Ala Gly Met Val Tyr Leu Ala Ser Leu His Phe Val His
        195                 200                 205

Arg Asp Leu Ala Thr Arg Asn Cys Leu Val Gly Gln Gly Leu Val Val
    210                 215                 220

Lys Ile Gly Asp Phe Gly Met Ser Arg Asp Ile Tyr Ser Thr Asp Tyr
225                 230                 235                 240

Tyr Arg Val Gly Gly Arg Thr Met Leu Pro Ile Arg Trp Met Pro Pro
                245                 250                 255

Glu Ser Ile Leu Tyr Arg Lys Phe Ser Thr Glu Ser Asp Val Trp Ser

260                 265                 270

Phe Gly Val Val Leu Trp Glu Ile Phe Thr Tyr Gly Lys Gln Pro Trp
        275                 280                 285

Tyr Gln Leu Ser Asn Thr Glu Ala Ile Glu Cys Ile Thr Gln Gly Arg
    290                 295                 300

Glu Leu Glu Arg Pro Arg Ala Cys Pro Pro Asp Val Tyr Ala Ile Met
305                 310                 315                 320

Arg Gly Cys Trp Gln Arg Glu Pro Gln Gln Arg Leu Ser Met Lys Asp
                325                 330                 335

Val His Ala Arg Leu Gln Ala Leu Ala Gln Ala Pro Pro Ser Tyr Leu
            340                 345                 350

Asp Val Leu Gly
        355

<210>  7
<211>  1068
<212>  DNA
<213>  Mus musculus

<400>  7
aaatgtggac agaggagcaa atttgggatc aaccgccctg ctgtattagc tccagaggat      60

gggctggcca tgtccctaca cttcatgaca ctgggtggca gttctctttc ccctactgag     120

ggcaaaggct ccggactcca gggccacatc atggagaacc cacagtactt cagtgatacc     180

tgtgtccatc acatcaagcg ccaggacatc attctcaagt gggagctagg ggagggagcc     240

tttggaaagg tctttctcgc tgagtgctac aaccttctga atgatcagga caagatgctc     300

gtggctgtca aggcactgaa ggaggcatcc gagaatgctc ggcaggactt tcagcgtgag     360

gccgagctgc tcaccatgct acagcaccag cacatcgtgc gcttctttgg agtctgcacc     420

gagggtggcc cgttgctcat ggtctttgag tacatgcgcc acggggacct caaccgtttc     480

ctccggtccc acggacctga tgcaaagctg ctggctggtg gtgaggatgt ggctcctggt     540

cctctgggcc ttgggcagct tctggctgtg gctagccagg tggctgctgg tatggtatat     600

ctagccagcc tgcactttgt gcaccgggat ctggccacac gcaactgtct ggtgggtcag     660

ggactagtgg tgaagattgg agactttggc atgagcagag acatctatag cacagactat     720

taccgagtgg gaggtcggac catgctgccc atccgctgga tgccacccga gagcatcctc     780

taccgcaagt tcagcaccga gagcgatgtg tggagctttg gggtggtgct ctgggagatc     840

ttcacctatg gaaagcagcc ctggtaccag ctctccaaca ctgaggcgat cgagtgtatc     900

acgcagggcc gggagctgga gcggccgcgc gcctgccctc ctgatgtcta cgccatcatg 960

cgaggctgct ggcagcgaga accgcagcaa cgcctcagca tgaaggatgt gcacgcgcgt 1020

ctgcaggccc tggcacaggc gccacccagt tacctggacg ttctgggc 1068

<210> 8
<211> 1182
<212> DNA
<213> Artificial Sequence

<220>
<223> nucleic acid sequence coding for CA-TrekA

<400> 8
atggcatcaa tgcagaagct gatctcagag gaggacctgg tcgacaaatg tggacagagg 60

agcaaatttg ggatcaaccg ccctgctgta ttagctccag aggatgggct ggccatgtcc 120

ctacacttca tgacactggg tggcagttct ctttccccta ctgagggcaa aggctccgga 180

ctccagggcc acatcatgga gaacccacag tacttcagtg atacctgtgt ccatcacatc 240

aagcgccagg acatcattct caagtgggag ctaggggagg gagcctttgg aaaggtcttt 300

ctcgctgagt gctacaacct tctgaatgat caggacaaga tgctcgtggc tgtcaaggca 360

ctgaaggagg catccgagaa tgctcggcag gactttcagc gtgaggccga gctgctcacc 420

atgctacagc accagcacat cgtgcgcttc tttggagtct gcaccgaggg tggcccgttg 480

ctcatggtct ttgagtacat gcgccacggg gacctcaacc gtttcctccg gtcccacgga 540

cctgatgcaa agctgctggc tggtggtgag gatgtggctc ctggtcctct gggccttggg 600

cagcttctgg ctgtggctag ccaggtggct gctggtatgg tatatctagc cagcctgcac 660

tttgtgcacc gggatctggc cacacgcaac tgtctggtgg gtcagggact agtggtgaag 720

attggagact ttggcatgag cagagacatc tatagcacag actattaccg agtgggaggt 780

cggaccatgc tgcccatccg ctggatgcca cccgagagca tcctctaccg caagttcagc 840

accgagagcg atgtgtggag ctttggggtg gtgctctggg agatcttcac ctatggaaag 900

cagccctggt accagctctc caacactgag gcgatcgagt gtatcacgca gggccgggag 960

ctggagcggc cgcgcgcctg ccctcctgat gtctacgcca tcatgcgagg ctgctggcag 1020

cgagaaccgc agcaacgcct cagcatgaag gatgtgcacg cgcgtctgca ggccctggca 1080

caggcgccac ccagttacct ggacgttctg ggctctagag ccatgagcaa agatggtaaa 1140

aagaagaaaa agaagtcaaa gacaaagtgt gtaattatgt aa 1182

<210> 9
<211> 371
<212> PRT
<213> Mus musculus

<400> 9

Lys Tyr Gly Arg Arg Ser Lys Phe Gly Met Lys Gly Pro Val Ala Val
1               5                   10                  15

Ile Ser Gly Glu Glu Asp Ser Ala Ser Pro Leu His His Ile Asn His
            20                  25                  30

Gly Ile Thr Thr Pro Ser Ser Leu Asp Ala Gly Pro Asp Thr Val Val
        35                  40                  45

Ile Gly Met Thr Arg Ile Pro Val Ile Glu Asn Pro Gln Tyr Phe Arg
    50                  55                  60

Gln Gly His Asn Cys His Lys Pro Asp Thr Tyr Val Gln His Ile Lys
65                  70                  75                  80

Arg Arg Asp Ile Val Leu Lys Arg Glu Leu Gly Glu Gly Ala Phe Gly
                85                  90                  95

Lys Val Phe Leu Ala Glu Cys Tyr Asn Leu Ser Pro Thr Lys Asp Lys
            100                 105                 110

Met Leu Val Ala Val Lys Ala Leu Lys Asp Pro Thr Leu Ala Ala Arg
        115                 120                 125

Lys Asp Phe Gln Arg Glu Ala Glu Leu Leu Thr Asn Leu Gln His Glu
    130                 135                 140

His Ile Val Lys Phe Tyr Gly Val Cys Gly Asp Gly Asp Pro Leu Ile
145                 150                 155                 160

Met Val Phe Glu Tyr Met Lys His Gly Asp Leu Asn Lys Phe Leu Arg
                165                 170                 175

Ala His Gly Pro Asp Ala Met Ile Leu Val Asp Gly Gln Pro Arg Gln
            180                 185                 190

Ala Lys Gly Glu Leu Gly Leu Ser Gln Met Leu His Ile Ala Ser Gln
        195                 200                 205

Ile Ala Ser Gly Met Val Tyr Leu Ala Ser Gln His Phe Val His Arg
    210                 215                 220

Asp Leu Ala Thr Arg Asn Cys Leu Val Gly Ala Asn Leu Leu Val Lys
225                 230                 235                 240

Ile Gly Asp Phe Gly Met Ser Arg Asp Val Tyr Ser Thr Asp Tyr Tyr
                245                 250                 255

Arg Val Gly Gly His Thr Met Leu Pro Ile Arg Trp Met Pro Pro Glu
            260                 265                 270

Ser Ile Met Tyr Arg Lys Phe Thr Thr Glu Ser Asp Val Trp Ser Phe
        275                 280                 285

Gly Val Ile Leu Trp Glu Ile Phe Thr Tyr Gly Lys Gln Pro Trp Phe
    290                 295                 300

Gln Leu Ser Asn Thr Glu Val Ile Glu Cys Ile Thr Gln Gly Arg Val
305                 310                 315                 320

Leu Glu Arg Pro Arg Val Cys Pro Lys Glu Val Tyr Asp Val Met Leu
                325                 330                 335

Gly Cys Trp Gln Arg Glu Pro Gln Gln Arg Leu Asn Ile Lys Glu Ile
            340                 345                 350

Tyr Lys Ile Leu His Ala Leu Gly Lys Ala Thr Pro Ile Tyr Leu Asp
        355                 360                 365

Ile Leu Gly
    370

<210> 10
<211> 1113
<212> DNA
<213> Mus musculus

<400> 10
aagtatggtc gccggtccaa atttggaatg aagggtcctg tggctgttat cagtggagag 60

gaggactcag ccagcccact gcatcacatc aaccatggca tcactacacc atcatcgttg 120

gatgctgggc ctgacacagt ggtcattggc atgacccgca tcccagtcat tgagaacccc 180

cagtacttcc gtcagggtca caattgccac aagccagaca catatgttca gcacatcaag 240

aggagagaca tcgtgttgaa gagagaattg ggtgagggag cctttgggaa ggtcttcctg 300

gctgagtgct acaatctaag ccccaccaaa gacaagatgc tagtggcagt gaaggccctg 360

aaggatccca ccttggctgc caggaaggat ttccagaggg aggctgagct gctcacgaac 420

ctgcagcatg agcatattgt caagttctat ggggtgtgtg gtgatggtga cccactcatc 480

atggtctttg aatacatgaa gcatggagac cttaacaagt tcctcagggc ccatggccca 540

gatgccatga tcctcgtgga tggacagcca cgtcaggcca agggggagct agggctctct 600

cagatgctcc acatcgccag tcagatagcc tcgggcatgg tgtacctggc ttcccagcac 660

tttgtacacc gggacctggc cacgaggaac tgcctggttg gagccaatct actagtgaag 720

attggagatt ttggcatgtc cagggacgtc tacagtactg attactacag ggtgggagga 780

cacaccatgc tccccatccg ctggatgccc cctgaaagca taatgtaccg gaagttcacc 840

acagagagtg atgtctggag cttcggggtt attctttggg agatctttac ctatggaaag 900

caaccatggt tccagctttc caacacggag gtcattgaat gcatcaccca aggccgtgtc 960

ttggagagac ccagagtctg ccctaaagaa gtgtatgatg tcatgctggg gtgctggcag 1020

agggaaccac agcagcggct gaatattaag gagatctaca aaatcctcca tgctttgggg 1080

aaggccaccc cgatctacct ggacattctt ggc 1113

<210> 11
<211> 1227
<212> DNA
<213> Artificial Sequence

<220>
<223> nucleic acid sequence coding for CA-TrkC

<400> 11
atggcatcaa tgcagaagct gatctcagag gaggacctgg tcgacaagta tggtcgccgg 60

tccaaatttg gaatgaaggg tcctgtggct gttatcagtg gagaggagga ctcagccagc 120

ccactgcatc acatcaacca tggcatcact acaccatcat cgttggatgc tgggcctgac 180

acagtggtca ttggcatgac ccgcatccca gtcattgaga acccccagta cttccgtcag 240

ggtcacaatt gccacaagcc agacacatat gttcagcaca tcaagaggag agacatcgtg 300

ttgaagagag aattgggtga gggagccttt gggaaggtct tcctggctga gtgctacaat 360

ctaagcccca ccaaagacaa gatgctagtg gcagtgaagg ccctgaagga tcccaccttg 420

gctgccagga aggatttcca gagggaggct gagctgctca cgaacctgca gcatgagcat 480

attgtcaagt tctatggggt gtgtggtgat ggtgacccac tcatcatggt ctttgaatac 540

atgaagcatg gagaccttaa caagttcctc agggcccatg ggccagatgc catgatcctc 600

gtggatggac agccacgtca ggccaagggg gagctagggc tctctcagat gctccacatc 660

gccagtcaga tagcctcggg catggtgtac ctggcttccc agcactttgt acaccgggac 720

ctggccacga ggaactgcct ggttggagcc aatctactag tgaagattgg agattttggc 780

atgtccaggg acgtctacag tactgattac tacagggtgg gaggacacac catgctcccc 840

atccgctgga tgccccctga aagcataatg taccggaagt tcaccacaga gagtgatgtc 900

tggagcttcg gggttattct ttgggagatc tttacctatg ggaagcaacc atggttccag 960

ctttccaaca cggaggtcat tgaatgcatc acccaaggcc gtgtcttgga gagacccaga 1020

gtctgcccta aagaagtgta tgatgtcatg ctggggtgct ggcagaggga accacagcag 1080

cggctgaata ttaaggagat ctacaaaatc ctccatgctt tggggaaggc caccccgatc 1140

tacctggaca ttcttggctc tagagccatg agcaaagatg gtaaaaagaa gaaaaagaag     1200

tcaaagacaa agtgtgtaat tatgtaa                                         1227

<210>  12
<211>  357
<212>  PRT
<213>  Homo sapiens

<400>  12

Lys Cys Gly Arg Arg Asn Lys Phe Gly Ile Asn Arg Pro Ala Val Leu
1               5                   10                  15

Ala Pro Glu Asp Gly Leu Ala Met Ser Leu His Phe Met Thr Leu Gly
            20                  25                  30

Gly Ser Ser Leu Ser Pro Thr Glu Gly Lys Gly Ser Gly Leu Gln Gly
        35                  40                  45

His Ile Ile Glu Asn Pro Gln Tyr Phe Ser Asp Ala Cys Val His His
    50                  55                  60

Ile Lys Arg Arg Asp Ile Val Leu Lys Trp Glu Leu Gly Glu Gly Ala
65                  70                  75                  80

Phe Gly Lys Val Phe Leu Ala Glu Cys His Asn Leu Leu Pro Glu Gln
                85                  90                  95

Asp Lys Met Leu Val Ala Val Lys Ala Leu Lys Glu Ala Ser Glu Ser
            100                 105                 110

Ala Arg Gln Asp Phe Gln Arg Glu Ala Glu Leu Leu Thr Met Leu Gln
        115                 120                 125

His Gln His Ile Val Arg Phe Phe Gly Val Cys Thr Glu Gly Arg Pro
    130                 135                 140

Leu Leu Met Val Phe Glu Tyr Met Arg His Gly Asp Leu Asn Arg Phe
145                 150                 155                 160

Leu Arg Ser His Gly Pro Asp Ala Lys Leu Leu Ala Gly Gly Glu Asp
                165                 170                 175

Val Ala Pro Gly Pro Leu Gly Leu Gly Gln Leu Leu Ala Val Ala Ser
            180                 185                 190

Gln Val Ala Ala Gly Met Val Tyr Leu Ala Gly Leu His Phe Val His
        195                 200                 205

Arg Asp Leu Ala Thr Arg Asn Cys Leu Val Gly Gln Gly Leu Val Val
    210                 215                 220

Lys Ile Gly Asp Phe Gly Met Ser Arg Asp Ile Tyr Ser Thr Asp Tyr
225                 230                 235                 240

Tyr Arg Val Gly Gly Arg Thr Met Leu Pro Ile Arg Trp Met Pro Pro
                245                 250                 255

Glu Ser Ile Leu Tyr Arg Lys Phe Thr Thr Glu Ser Asp Val Trp Ser
            260                 265                 270

Phe Gly Val Val Leu Trp Glu Ile Phe Thr Tyr Gly Lys Gln Pro Trp
        275                 280                 285

Tyr Gln Leu Ser Asn Thr Glu Ala Ile Asp Cys Ile Thr Gln Gly Arg
    290                 295                 300

Glu Leu Glu Arg Pro Arg Ala Cys Pro Pro Glu Val Tyr Ala Ile Met
305                 310                 315                 320

Arg Gly Cys Trp Gln Arg Glu Pro Gln Gln Arg His Ser Ile Lys Asp
                325                 330                 335

Val His Ala Arg Leu Gln Ala Leu Ala Gln Ala Pro Pro Val Tyr Leu
            340                 345                 350

Asp Val Leu Gly Glx
        355

<210>  13
<211>  1071
<212>  DNA
<213>  Homo sapiens

<400>  13
aaatgtggac ggagaaacaa gtttgggatc aaccgcccgg ctgtgctggc tccagaggat      60

gggctggcca tgtccctgca tttcatgaca ttgggtggca gctccctgtc ccccaccgag     120

ggcaaaggct ctgggctcca aggccacatc atcgagaacc cacaatactt cagtgatgcc     180

tgtgttcacc acatcaagcg ccgggacatc gtgctcaagt gggagctggg ggagggcgcc     240

tttgggaagg tcttccttgc tgagtgccac aacctcctgc ctgagcagga caagatgctg     300

gtggctgtca aggcactgaa ggaggcgtcc gagagtgctc ggcaggactt ccagcgtgag     360

gctgagctgc tcaccatgct gcagcaccag cacatcgtgc gcttcttcgg cgtctgcacc     420

gagggccgcc ccctgctcat ggtctttgag tatatgcggc acggggacct caaccgcttc     480

ctccgatccc atggacctga tgccaagctg ctggctggtg gggaggatgt ggctccaggc 540

cccctgggtc tggggcagct gctggccgtg gctagccagg tcgctgcggg gatggtgtac 600

ctggcgggtc tgcattttgt gcaccgggac ctggccacac gcaactgtct agtgggccag 660

ggactggtgg tcaagattgg tgattttggc atgagcaggg atatctacag caccgactat 720

taccgtgtgg gaggccgcac catgctgccc attcgctgga tgccgcccga gagcatcctg 780

taccgtaagt tcaccaccga gagcgacgtg tggagcttcg gcgtggtgct ctgggagatc 840

ttcacctacg gcaagcagcc ctggtaccag ctctccaaca cggaggcaat cgactgcatc 900

acgcagggac gtgagttgga gcggccacgt gcctgcccac cagaggtcta cgccatcatg 960

cggggctgct ggcagcggga gccccagcaa cgccacagca tcaaggatgt gcacgcccgg 1020

ctgcaagccc tggcccaggc acctcctgtc tacctggatg tcctgggcta g 1071

<210> 14
<211> 372
<212> PRT
<213> Homo sapiens

<400> 14

Lys Tyr Gly Arg Arg Ser Lys Phe Gly Met Lys Gly Pro Val Ala Val
1               5                   10                  15

Ile Ser Gly Glu Glu Asp Ser Ala Ser Pro Leu His His Ile Asn His
            20                  25                  30

Gly Ile Thr Thr Pro Ser Ser Leu Asp Ala Gly Pro Asp Thr Val Val
        35                  40                  45

Ile Gly Met Thr Arg Ile Pro Val Ile Glu Asn Pro Gln Tyr Phe Arg
    50                  55                  60

Gln Gly His Asn Cys His Lys Pro Asp Thr Tyr Val Gln His Ile Lys
65                  70                  75                  80

Arg Arg Asp Ile Val Leu Lys Arg Glu Leu Gly Glu Gly Ala Phe Gly
                85                  90                  95

Lys Val Phe Leu Ala Glu Cys Tyr Asn Leu Ser Pro Thr Lys Asp Lys
            100                 105                 110

Met Leu Val Ala Val Lys Ala Leu Lys Asp Pro Thr Leu Ala Ala Arg
        115                 120                 125

Lys Asp Phe Gln Arg Glu Ala Glu Leu Leu Thr Asn Leu Gln His Glu
    130                 135                 140

His Ile Val Lys Phe Tyr Gly Val Cys Gly Asp Gly Asp Pro Leu Ile
145                 150                 155                 160

Met Val Phe Glu Tyr Met Lys His Gly Asp Leu Asn Lys Phe Leu Arg
                165                 170                 175

Ala His Gly Pro Asp Ala Met Ile Leu Val Asp Gly Gln Pro Arg Gln
            180                 185                 190

Ala Lys Gly Glu Leu Gly Leu Ser Gln Met Leu His Ile Ala Ser Gln
        195                 200                 205

Ile Ala Ser Gly Met Val Tyr Leu Ala Ser Gln His Phe Val His Arg
    210                 215                 220

Asp Leu Ala Thr Arg Asn Cys Leu Val Gly Ala Asn Leu Leu Val Lys
225                 230                 235                 240

Ile Gly Asp Phe Gly Met Ser Arg Asp Val Tyr Ser Thr Asp Tyr Tyr
                245                 250                 255

Arg Val Gly Gly His Thr Met Leu Pro Ile Arg Trp Met Pro Pro Glu
            260                 265                 270

Ser Ile Met Tyr Arg Lys Phe Thr Thr Glu Ser Asp Val Trp Ser Phe
        275                 280                 285

Gly Val Ile Leu Trp Glu Ile Phe Thr Tyr Gly Lys Gln Pro Trp Phe
    290                 295                 300

Gln Leu Ser Asn Thr Glu Val Ile Glu Cys Ile Thr Gln Gly Arg Val
305                 310                 315                 320

Leu Glu Arg Pro Arg Val Cys Pro Lys Glu Val Tyr Asp Val Met Leu
                325                 330                 335

Gly Cys Trp Gln Arg Glu Pro Gln Gln Arg Leu Asn Ile Lys Glu Ile
            340                 345                 350

Tyr Lys Ile Leu His Ala Leu Gly Lys Ala Thr Pro Ile Tyr Leu Asp
        355                 360                 365

Ile Leu Gly Glx
    370

<210>  15
<211>  1116

<212>  DNA
<213>  Homo sapiens

<400>  15
aaatatggtc gacggtccaa atttggaatg aagggtcccg tggctgtcat cagtggtgag      60

gaggactcag ccagcccact gcaccacatc aaccacggca tcaccacgcc ctcgtcactg     120

gatgccgggc ccgacactgt ggtcattggc atgactcgca tccctgtcat tgagaacccc     180

cagtacttcc gtcagggaca caactgccac aagccggaca cgtatgtgca gcacattaag     240

aggagagaca tcgtgctgaa gcgagaactg ggtgagggag cctttggaaa ggtcttcctg     300

gccgagtgct acaacctcag cccgaccaag gacaagatgc ttgtggctgt gaaggccctg     360

aaggatccca ccctggctgc ccggaaggat ttccagaggg aggccgagct gctcaccaac     420

ctgcagcatg agcacattgt caagttctat ggagtgtgcg gcgatgggga ccccctcatc     480

atggtctttg aatacatgaa gcatggagac ctgaataagt tcctcagggc ccatgggcca     540

gatgcaatga tccttgtgga tggacagcca cgccaggcca agggtgagct ggggctctcc     600

caaatgctcc acattgccag tcagatcgcc tcgggtatgg tgtacctggc ctcccagcac     660

tttgtgcacc gagacctggc caccaggaac tgcctggttg gagcgaatct gctagtgaag     720

attggggact tcggcatgtc cagagatgtc tacagcacgg attattacag ggtgggagga     780

cacaccatgc tccccattcg ctggatgcct cctgaaagca tcatgtaccg gaagttcact     840

acagagagtg atgtatggag cttcggggtg atcctctggg agatcttcac ctatggaaag     900

cagccatggt tccaactctc aaacacggag gtcattgagt gcattaccca aggtcgtgtt     960

ttggagcggc cccgagtctg ccccaaagag gtgtacgatg tcatgctggg gtgctggcag    1020

agggaaccac agcagcggtt gaacatcaag gagatctaca aaatcctcca tgctttgggg    1080

aaggccaccc caatctacct ggacattctt ggctag                              1116